# EUROPEAN PATENT SPECIFICATION

(11) **EP 2 170 388 B1**
(45) Date of publication and mention of the grant of the patent: **12.10.2016**
(21) Application number: 08781323.4
(22) Date of filing: 03.07.2008
(51) Int. Cl.: C07K 16/00, A61K 39/395, C07K 16/28, A61K 9/00

(54) **ANTI-CD20 ANTIBODY FORMULATIONS**
ANTI-CD20 ANTIKÖRPER-FORMULIERUNGEN
FORMULATIONS D'ANTICORPS ANTI-CD20

(30) Priority: 06.07.2007 US 948220 P
(43) Date of publication of application: 07.04.2010
(60) Divisional application: 15155712.1
(73) Proprietor: Novartis AG, 4056 Basel (CH)
(72) Inventor: BRISBANE, Charlene, E., King of Prussia, PA 19406 (US); KETKAR, Amol, Sharad, King of Prussia, PA 19406 (US); LASHMAR, Ulla, Tove, Harlow, Essex CM19 5AW (GB)
(74) Representative: Pfister-Fu, Yixin
(86) International application number: PCT/US2008/069125
(87) International publication number: WO 2009/009407

(56) References cited:
- WO-A1-98/56418
- WO-A1-2009/009406
- WO-A2-2004/001007
- WO-A2-2007/036745
- US-A- 4 816 401
- US-A1- 2006 093 598
- US-A1- 2006 121 032
- US-A1- 2006 246 004
- US-A1- 2006 246 004
- Summary of product characteristics - MabThera/rituximab -
- 'Arzerra', [Online] Retrieved from the Internet: <URL:health.gsk> [retrieved on 2012-11-16]
- Rituximab
- CHMP assessment report for Arzerra

## Description

### FIELD OF THE INVENTION

This invention relates to shear and the temperature stable antibody formulations.

### BACKGROUND OF THE INVENTION

Proteins are larger and more complex than traditional organic and inorganic drugs (i.e. possessing multiple functional groups in addition to complex three-dimensional structures), and the formulation of such proteins poses special problems. For a protein to remain biologically active, a formulation must preserve the intact conformational integrity of at least a core sequence of the protein's amino acids while at the same time protecting the protein's multiple functional groups from degradation. Degradation pathways for proteins can involve chemical instability (i.e. any process which involves modification of the protein by bond formation of cleavage resulting in a new chemical entity) or physical instability (i.e. changes in the higher order structure of the protein). Chemical instability can result from deamidation, racemization, hydrolysis, oxidation, beta elimination or disulfide exchange. Physical instability can result from denaturation, aggregation, precipitation or adsorption, for example. The three most common protein degradation pathways are protein aggregation, deamidation and oxidation. Cleland et al. Critical Reviews in Therapeutic Drug Carrier Systems 10(4): 307-377 (1993).

The CD20 molecule (also called human B-lymphocyte-restricted differentiation antigen or Bp35) is a hydrophobic transmembrane protein with a molecular weight of approximately 35 kD located on pre-B and mature B lymphocytes (Valentine et al. (1989) J. Biol. Chem. 264(19): 11282-11287; and Einfield et al. (1988) EMBO J. 7(3):711-717). CD20 is found on the surface of greater than 90% of B cells from peripheral blood or lymphoid organs and is expressed during early pre-B cell development and remains until plasma cell differentiation. CD20 is present on both normal B cells as well as malignant B cells. In particular, CD20 is expressed on greater than 90% of B cell non-Hodgkin's lymphomas (NHL) (Anderson et al. (1984) Blood 63(6):1424-1433), but is not found on hematopoietic stem cells, pro-B cells, normal plasma cells, or other normal tissues (Tedder et al. (1985) J. Immunol. 135(2):973-979).

The 85 amino acid carboxyl-terminal region of the CD20 protein is located within the cytoplasm. The length of this region contrasts with that of other B cell-specific surface structures such as IgM, IgD, and IgG heavy chains or histocompatibility antigens class II .alpha. or .beta. chains, which have relatively short intracytoplasmic regions of 3, 3, 28, 15, and 16 amino acids, respectively (Komaromy et al. (1983) NAR 11:6775-6785). Of the last 61 carboxyl-terminal amino acids, 21 are acidic residues, whereas only 2 are basic, indicating that this region has a strong net negative charge. The GenBank Accession No. is NP.sub.--690605.

It is thought that CD20 might be involved in regulating an early step(s) in the activation and differentiation process of B cells (Tedder et al. (1986) Eur.J. Immunol. 16:881-887) and could function as a calcium ion channel (Tedder et al. (1990) J. Cell. Biochem. 14D: 195).

Despite uncertainty about the actual function of CD20 in promoting proliferation and/or differentiation of B cells, it provides an important target for antibody mediated therapy to control or kill B cells involved in cancers and autoimmune disorders. In particular, the expression of CD20 on tumor cells, e.g., NHL, makes it an important target for antibody mediated therapy to specifically target therapeutic agents against CD20-positive neoplastic cells. US 2006/0246004 A1 discloses a liquid multidose formulation comprising hu2H7 antibody at 40 mg/ml, a humanized antibody that binds human CD20, 2.5 mM acetate, 150 mM trehalose, 0.9% benzyl alcohol, 0.02% polysorbate 20 at pH of 5.0 that has a minimum shelf life of two years storage at 2-8° C and that can be used in the treatment of B cell malignancies and autoimmune diseases.

HuMax-CD20™ (ofatumumab), described as 2F2 antibody in WO2004/035607, is a fully human IgG1,K high-affinity antibody targeted at the CD20 molecule in the cell membrane of B-cells. HuMax-CD20™ is in clinical development for the treatment of non-Hodgkin's lymphoma (NHL), chronic lymphocytic leukemia (CLL), and rheumatoid arthritis (RA). See also Teeling et al., Blood, 104, pp 1793 (2004); and Teeling et al., J. Immunology, 177, pp 362-371 (2007).

There is a need for formulating a shear and temperature stable pharmaceutical formulation comprising an antibody which is suitable for therapeutic use. In one embodiment the antibody can be a monoclonal antibody. In another embodiment the antibody can be an anti-CD20 antibody, including but not limited to ofatumumab, rituximab, tositumomab, ocrelizumab (2H7.v16), 11B8 or 7D8 (disclosed in WO2004/035607), an anti-CD20 antibody disclosed in WO 2005/103081 such as C6, an anti-CD antibody disclosed in WO2003/68821 such as IMMU-106 (from Immunomedics), an anti-CD20 antibody disclosed in WO2004/103404 such as AME-133 (from Applied Molecular Evolution/Lilly), and anti-CD20 antibody disclosed in US 2003/0118592 such as TRU-015 (from Trubion Pharmaceuticals Inc).

### SUMMARY OF THE INVENTION

The present invention relates to a shear and temperature stable aqueous antibody formulation.

In one aspect, the invention relates to an ofatumumab formulation comprising a therapeutically effective amount of ofatumumab, wherein the formulation further comprises 10 to 100 mM sodium acetate, 25 to 100 mM sodium chloride, 0.5 to 5% arginine free base, 0.02 to 0.2 mM EDTA, 0.01 to 0.2 % polysorbate 80 and adjusted to pH 5.0 to 7.0.

In one embodiment, the invention relates to an ofatumumab formulation comprising an ofatumumab in the concentration range of 20-300 mg/mL, wherein the formulation further comprises 50 mM sodium acetate, 51 mM sodium chloride, 1% arginine free base, 0.05 mM EDTA, 0.02% polysorbate 80, and adjusted to pH 5.5.

In yet another embodiment, the invention relates to an anti-CD20 antibody formulation wherein the formulation is stable for at least 2 years. In another embodiment, the invention relates to an anti-CD20 antibody formulation wherein the formulation is stable at temperatures up to at least 55°C. In another embodiment, the invention relates to an anti-CD20 antibody formulation wherein the formulation is stable at a temperature of about 5°C for at least 2 years. In another embodiment, the invention relates to an anti-CD20 antibody formulation wherein the formulation is stable at a temperature of about 25°C for at least 3 months. In another embodiment, the invention relates to an anti-CD20 antibody formulation wherein the formulation is stable at a temperature of about 40°C for at least 1 month. In another embodiment, the invention relates to an anti-CD20 antibody formulation wherein the formulation is stable at a temperature of about 55°C for at least 1 day. In another embodiment, the invention relates to an anti-CD20 antibody formulation wherein the formulation is stable at a temperature range of approximately, 5 to 25°C, 5 to 35°C, 5 to 45°C, 10 to 25°C, 10 to 35°C, 10 to 45°C, 10 to 55°C, 20 to 35°C, 20 to 45°C, or 20 to 55°C for at least 1 day with shaking..

In another embodiment, the invention relates to an ofatumumab formulation wherein the antibody is present in an amount of about 20-300 mg/mL, 50-300 mg/mL, 100-300 mg/mL, 150-300 mg/mL, 200-300 mg/mL, or 250-300 mg/mL.

In another embodiment, the invention relates to an anti-CD20 antibody formulation wherein sodium acetate is present in an amount of about 50mM, 40mM, 45mM, 55mM, or 60mM. In other embodiments, the sodium acetate may be present in an amount of 10 to 100 mM, 20 to 100 mM, 30 to 100 mM, 40 to 100 mM, 50 to 100 mM, 60 to 100 mM, 70 to 100 mM, 25 to 80 mM, or 30 to 70 mM.

In yet another embodiment, the invention relates to an ofatumumab formulation wherein acetic acid is present (about 100 mM acetic acid) to adjust the formulation to about pH 5.5. In other embodiments, the pH may be adjusted to pH 5.0, 5.5, 6.0, 6.5 or 7.0. In yet other embodiments of the invention, NaOH or HCl is used to adjust the pH to 5.0, 5.5, 6.0, 6.5 or 7.0.

In yet another embodiment, the invention relates to an ofatumumab formulation wherein sodium chloride is present in an amount of about 51mM, 45mM, 46mM, 47mM, 48mM, 49mM, 50mM, 52mM, 53mM, 54mM, 55mM. In other embodiments, the sodium chloride may be present in an amount of 25 to 100 mM, 35 to 90 mM, 45 to 80 mM, 25 to 70 mM, or 45 to 70 mM.

In another embodiment, the invention relates to an ofatumumab formulation wherein arginine free base is present in an amount of about 1%, 0.7%, 1.3%, or 2.0%. In other embodiments, the arginine free base may be between 0.5 and 5.0%, 0.5 to 2.0%, 0.5 to 2.5%, 0.5 to 3.0%, 0.5 to 3.5%, 0.5 to 4.0%, or 0.5 to 4.5%.

In another embodiment, the invention relates to an ofatumumab formulation wherein EDTA is present in an amount of about 0.05 mM, 0.03 mM, 0.04 mM, or 0.06 mM. In other embodiments, the EDTA may be present in an amount of 0.02 mM - 0.2 mM, 0.02 mM - 0.1 mM, 0.02 mM - 0.15 mM, 0.04 mM - 0.1 mM, 0.03 mM - 0.15 mM, or 0.03 mM - 0.2 mM.

In another embodiment, the invention relates to an ofatumumab formulation wherein polysorbate 80 is present in an amount of about 0.02%, 0.015%, or 0.025%. In other embodiments, the polysorbate 80 may be present in an amount of 0.01 - 0.2%, 0.01 - 0.15%, 0.02 - 0.2%, 0.02 - 0.15%, 0.01 - 0.25%, or 0.01 - 0.05%.

Described is a method of treating a disease involving cells expressing CD20 by administering to a mammal ofatumumab formulation of the present invention comprising a therapeutically effective amount of ofatumumab, wherein the formulation further comprises 10 to 100 mM sodium acetate, 25 to 100 mM sodium chloride, 0.5 to 5% arginine free base, 0.02 to 0.2 mM EDTA, 0.01 to 0.2 % polysorbate 80 and adjusted to pH 5.0 to 7.0. Exemplary "diseases involving cells expressing CD20" that can be treated (e.g., ameliorated) or prevented include, but are not limited to, tumorigenic diseases and immune diseases, e.g., autoimmune diseases. Examples of tumorigenic diseases which can be treated and/or prevented include B cell lymphoma, e.g., NHL, including precursor B cell lymphoblastic leukemia/lymphoma and mature B cell neoplasms, such as B cell chronic lymhocytic leukemia (CLL)/small lymphocytic lymphoma (SLL), B cell prolymphocytic leukemia, lymphoplasmacytic lymphoma, mantle cell lymphoma (MCL), follicular lymphoma (FL), including low-grade, intermediate-grade and high-grade FL, cutaneous follicle center lymphoma, marginal zone B cell lymphoma (MALT type, nodal and splenic type), hairy cell leukemia, diffuse large B cell lymphoma, Burkitt's lymphoma, plasmacytoma, plasma cell myeloma, post-transplant lymphoproliferative disorder, Waldenstrom's macroglobulinemia, and anaplastic large-cell lymphoma (ALCL). Examples of immune disorders in which CD20 expressing B cells are involved which can be treated and/or prevented include psoriasis, psoriatic arthritis, dermatitis, systemic scleroderma and sclerosis, inflammatory bowel disease (IBD), Crohn's disease, ulcerative colitis, respiratory distress syndrome, meningitis, encephalitis, uveitis, glomerulonephritis, eczema, asthma, atherosclerosis, leukocyte adhesion deficiency, multiple sclerosis, Raynaud's syndrome, Sjogren's syndrome, juvenile onset diabetes, Reiter's disease, Behcet's disease, immune complex nephritis, IgA nephropathy, IgM polyneuropathies, immune-mediated thrombocytopenias, such as acute idiopathic thrombocytopenic purpura and chronic idiopathic thrombocytopenic purpura, hemolytic anemia, myasthenia gravis, lupus nephritis, systemic lupus erythematosus, rheumatoid arthritis (RA), atopic dermatitis, pemphigus, Graves' disease, Hashimoto's thyroiditis, Wegener's granulomatosis, Omenn's syndrome, chronic renal failure, acute infectious mononucleosis, HIV, and herpes virus associated diseases. Further examples are severe acute respiratory distress syndrome and choreoretinitis. Yet further examples are diseases and disorders caused by infection of B-cells with virus, such as Epstein-Barr virus (EBV). Yet a further example is COPD.

Described is a method of treating a disease involving cells expressing CD20 by administering to a mammal an ofatumumab formulation of the present invention comprising a therapeutically effective amount of ofatumumab, wherein the formulation further comprises 10 to 100 mM sodium acetate, 25 to 100 mM sodium chloride, 0.5 to 5% arginine free base, 0.02 to 0.2 mM EDTA, 0.01 to 0.2 % polysorbate 80 and adjusted to pH 5.0 to 7.0 and wherein the stable antibody formulation is administered orally, parenterally, intranasally, vaginally, rectally, lingually, sublingually, bucally, transdermally, intravenously, or subcutaneously to a mammal.

Described is a method of treating a disease involving cells expressing CD20 by administering to a mammal an ofatumumab formulation of the present invention comprising ofatumumab in the concentration range of 20-300 mg/mL, wherein the formulation further comprises 50 mM sodium acetate, 51 mM sodium chloride, 1% arginine free base, 0.05 mM EDTA, 0.02% polysorbate 80, and adjusted to pH 5.5.

It is to be understood that both the foregoing summary description and the following detailed description are exemplary and explanatory, and are intended to provide further explanation of the invention as claimed.

The accompanying drawings are included to provide a further understanding of the invention, and are incorporated in, and constitute a part of this specification, illustrate several embodiments of the invention, and together with the description serve to explain the principles of the invention.

### BRIEF DESCRIPTION OF THE FIGURES

Figure 1 illustrates the standard formulation (RefMat) of anti-CD20 antibody at 20 mg/mL (30 mM citrate, 100mM NaCl, pH 6.5) in duplicate.
Figure 2 illustrates one embodiment of the invention (PlatForm) formulation of anti-CD20 antibody at 20 mg/mL (50 mM sodium acetate, sodium chloride (51 mM), 1% arginine free base, 0.05 mM EDTA, 0.02% polysorbate 80, and adjusted to pH to 5.5 with HCl) in duplicate.
Figure 3 graphically illustrates a comparison of anti-CD20 antibody thermal stability in a formulation embodiment of the invention (PlatForm) and standard formulation buffers (RefMat) by DSC. Thermodynamically, the two formulations are similar as seen by their DSC profiles since the change in apparent Tm is less than 0.5°C between the formulations.

### DETAILED DESCRIPTION OF THE INVENTION

One embodiment of the present invention relates to shear and temperature stable antibody formulations.

In another embodiment, the invention provides for an unexpected stability seen for a formulation under simultaneous stress conditions of elevated temperature and shaking at 55°C.

A further embodiment of the invention is a more stable formulation than compared to a standard formulation (such as 30 mM citrate, 100mM NaCl, pH 6.5). The present invention's formulation showed reduced precipitation (remained clear) when subjected to stress conditions but the standard formulation had aggregated. This result was unpredictable because thermodynamically the two formulations are similar as seen by their DSC (differential scanning calorimeter) profiles.

In the description of the present invention, certain terms are used as defined below.

The term "protein formulation" or "antibody formulation" refers to preparations which are in such form as to permit the biological activity of the active ingredients to be unequivocally effective, and which contain no additional components which are toxic to the subjects to which the formulation would be administered.

"Pharmaceutically acceptable" excipients (vehicles, additives) are those which can reasonably be administered to a subject mammal to provide an effective dose of the active ingredient employed. For example, the concentration of the excipient is also relevant for acceptability for injection.

A "stable" formulation is one in which the protein therein essentially retains its physical and/or chemical stability and/or biological activity upon storage. Various analytical techniques for measuring protein stability are available in the art and are reviewed in Peptide and Protein Drug Delivery, 247-301, Vincent Lee Ed., Marcel Dekker, Inc., New York, N.Y., Pubs (1991) and Jones, A. Adv. Drug Delivery Rev. 10: 29-90 (1993), for example. Stability can be measured at a selected temperature for a selected time period. Preferably, the formulation is stable at ambient temperature or at 40°C for at least 1 month and/or stable at 2-8°C for at least 1 to 2 years. Furthermore, it is desirable that the formulation be stable following freezing (e.g. to - 70°C) and thawing of the product.

A protein "retains its physical stability" in a biopharmaceutical formulation if it shows little to no change in aggregation, precipitation and/or denaturation as observed by visual examination of color and/or clarity, or as measured by UV light scattering (measures visible aggregates) or size exclusion chromatography (SEC). SEC measures soluble aggregates that are not necessarily a precursor for visible aggregates.

A protein "retains its chemical stability" in a biopharmaceutical formulation, if the chemical stability at a given time is such that the protein is considered to retain its biological activity as defined below. Chemically degraded species may be biologically active and chemically unstable. Chemical stability can be assessed by detecting and quantifying chemically altered forms of the protein. Chemical alteration may involve size modification (e.g. clipping) which can be evaluated using SEC, SDS-PAGE and/or matrix-assisted laser desorption ionization/time-of-flight mass spectrometry (MALDI/TOF MS), for example. Other types of chemical alteration include charge alteration (e.g. occurring as a result of deamidation) which can be evaluated by ion-exchange chromatography, for example.

An antibody "retains its biological activity" in a pharmaceutical formulation, if the change in the biological activity of the antibody at a given time is within about 10% (within the errors of the assay) of the biological activity exhibited at the time the pharmaceutical formulation was prepared as determined in an antigen binding assay, for example. Other "biological activity" assays for antibodies are elaborated herein below.

The term "isotonic" means that the formulation of interest has essentially the same osmotic pressure as human blood. In one embodiment, the isotonic formulations of the invention will generally have an osmotic pressure in the range of 250 to 350 mOsm. In other embodiments, isotonic formulations of the invention will have an osmotic pressure from about 350 to 450 mOsm. In yet another embodiment, isotonic formulations of the invention will have an osmotic pressure above 450 mOsm. Isotonicity can be measured using a vapor pressure or ice-freezing type osmometer for example.

As used herein, "buffer" refers to a buffered solution that resists changes in pH by the action of its acid-base conjugate components. In one embodiment, the buffer of this invention has a pH in the range from about 4.5 to about 6.0; in another embodiment, from about 4.8 to about 5.8; and in a further embodiment, a pH of about 5.5. Examples of buffers that will control the pH in this range include acetate (e.g. sodium acetate), succinate (such as sodium succinate), gluconate, histidine, citrate and other organic acid buffers. Where a freeze-thaw stable formation is desired, the buffer is preferably not phosphate.

In a pharmacological sense, in the context of the present invention, a "therapeutically effective amount" of an antibody refers to an amount effective in the prevention or treatment of a disorder for the treatment of which the antibody is effective. A "disorder" is any condition that would benefit from treatment with the antibody. This includes chronic and acute disorders or diseases including those pathological conditions which predispose the mammal to the disorder in question. In a preferred embodiment "disorder" is a disease involving cells expressing CD20.

A "preservative" is a compound which can be included in the formulation to essentially reduce bacterial action therein, thus facilitating the production of a multi-use formulation, for example. Examples of potential preservatives include octadecyldimethylbenzyl ammonium choride, hexamethonium chloride, benzalkonium chloride (a mixture of alkylbenzyldimethylammonium chlorides in which the alkyl groups are long-chain compounds), and benzelthonium chloride. Other types of preservatives include aromatic alcohols such as phenol, butyl and benzyl alcohol, alkyl parabens such as methyl or propyl paraben, catechol, resorcinol, cyclohexanol, 3-pentanol, and m-cresol. The most preferred preservation herein is benzyl alcohol.

The term "antibody" is used in the broadest sense and specifically covers monoclonal antibodies (including full length monoclonal antibodies), polyclonal antibodies, multispecific antibodies (e.g. bispecific antibodies), and antibody fragments so long as they exhibit the desired biological activity.

"Antibody fragments" comprise a portion of a full length antibody, generally the antigen binding or variable region thereof. Examples of antibody fragments include Fab, Fab', F(ab')₂, and Fv fragments; diabodies; linear antibodies; single-chain antibody molecules; and multispecific antibodies formed from antibody fragments.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determination on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, e.g., U.S. Pat. No. 4,816,567). The "monoclonal antibodies" may also be isolated from phage antibody libraries using the technique described in Clackson et al., Nature 352:624-626 (1991) and Marks et al., J. Mol. Biol. 222:581-597 (1991), for example.

"Humanized" forms of non-human (e.g., murine) antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, FR residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

"Single-chain Fv" or "sFv" antibody fragments comprise the V_{H} and V_{L} domain of antibody, wherein these domains are present in a single polypeptide chain. Generally, the Fv polypeptide further comprises a polypeptide linker between the V_{H} and V_{L} domains which enables the SFv to form the desired structure for antigen binding. For a view of sFv see Pluckthun in The Pharmacology of Monoclonal Antibodies, vol. 113, Rosenburg and Moore eds. Springer-Verlag, N.Y., pp. 269-315 (1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H} and V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA 90:6444-6448 (1993).

The expression "linear antibodies" when used throughout the application refers to the antibodies described in Zapata et al. Protein Eng. 8(10):1057-1062 (1995). Briefly, these antibodies comprise a pair of tandem Fd segments (V_{H} --C_{H} --V _{H1} --C_{H1}) which form a pair of antigen binding regions. Linear antibodies can be bispecific or monospecific.

The antibody which is formulated is preferably essentially pure and desirably essentially homogenous (i.e. free from contaminating proteins etc). "Essentially pure" antibody means a composition comprising at least about 90% by weight of the antibody, based on total weight of the composition, preferably at least about 95% by weight. "Essentially homogeneous" antibody means a composition comprising at least about 99% by weight of the antibody, based on total weight of the composition.

"Treatment" refers to both therapeutic treatment and prophylactic or preventative measures. Those in need of treatment include those already with the disorder as well as those in which the disorder is to be prevented.

"Mammal" for purposes of treatment refers to any animal classified as a mammal, including but not limited to humans, domestic and farm animals, and zoo, sports, or pet animals, such as dogs, horses, cats, and cows.

"Stress condition" refers to an environment which is chemically and physically unfavorable for a protein and may render unacceptable protein stability (e.g. thermal, shear, chemical stress).

Size Exclusion Chromatography is a chromatographic method in which particles are separated based on their size or hydrodynamic volume.

Dynamic Light Scattering is a method which measures the time dependence of protein scattered light. Traditionally, this time dependence is processed to yield the hydrodynamic radius of a molecule.

"DSC" refers to differential scanning calorimeter: DSC acquisition parameters: can be but not limited to, 1 mg/ml protein, scan for 5 to 80°C with a scan rate of 70°C per hour and 15 minute prewait. A buffer-buffer scan can be acquired first and subtracted from the raw data. The data can be corrected for the buffer and normalized for the protein concentration then plotted. Aggregation can prevent baseline correction.

The following examples are further illustrative of the present invention. The examples are not intended to limit the scope of the present invention, and provide further understanding of the invention.

### EXAMPLES

The invention is further illustrated by way of the following examples which are intended to elucidate the invention. These examples are not intended, nor are they to be construed, as limiting the scope of the invention. The examples below are carried out using standard techniques, and such standard techniques are well known and routine to those of skill in the art, except where otherwise described in detail.

### Example 1.1: Preparation of the platform formulation buffer

In one embodiment of the invention, 4 liters of acetate buffer were prepared. In this embodiment, the final buffer was comprised of 50 mM sodium acetate, 0.05 mM EDTA, 51 mM NaCl, 1.0% Arginine, 0.02% Polysorbate 80, pH 5.5. The buffer was prepared by dissolving sodium actetate trihydrate, edetate disodium (EDTA), polysorbate 80 and L-arginine free base into 3.5L of deoinized water. Once the pH was adjusted to 5.5 using 3N HCl, the volume was brought up to 4.0L and the buffer was filtered using a 0.45 µm filter unit. The buffer can then be stored at 2-8°C until use. The formulation "%" described in the present application refers to "% by volume".

### Example 2.1: Preparation of ofatumumab in a platform formulation buffer

In one embodiment of the invention, ofatumumab was diafiltrated into a platform formulation (50 mM Sodium Acetate, 51 mM NaCl, 0.05mM EDTA, 0.02% Polysorbate 80, and 1.0% Arginine (free-base)) and concentrated for stability. Ofatumumab was diafiltrated in to the platform formulation using a lab-scale tangential flow system with three membranes. After the diafiltration into the platform buffer, ofatumumab was concentrated to a maximum concentration of 179 mg/mL. The entire process took approximately three working days to complete and the yield was 96.1 %. Some of the 179 mg/mL was diluted with platform formulation buffer so that a concentration range of ∼20 - 179 mg/mL could be studied.

### Example 3.1: Preparation of ofatumumab in Standard and Platform Formulation for General Appearance (GA) Direct Comparison

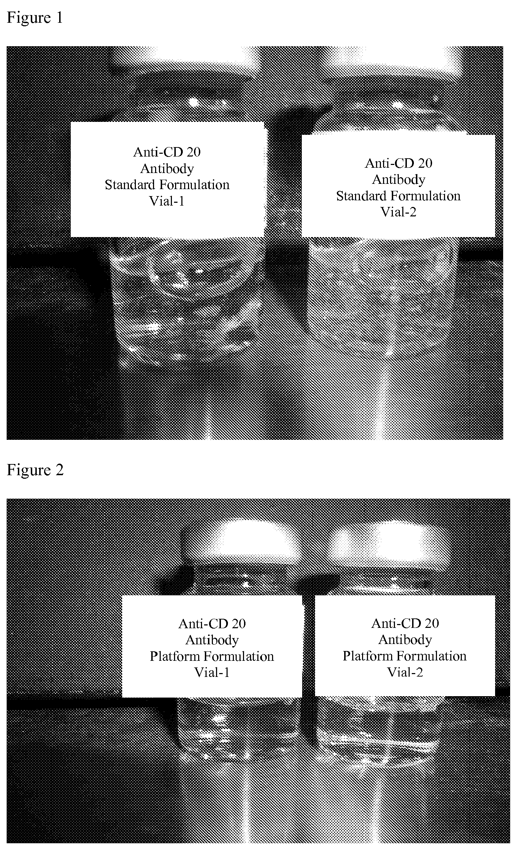

An anti-CD20 antibody (ofatumumab) was prepared in the standard formulation and the platform (one embodiment of the present invention) formulation at a concentration of 20 mg/mL for general appearance in direct comparison over a 12 week time period and for shake experiments. The anti-CD20 antibody in the standard and platform formulations were filtered using a low protein binding 0.2 µm membrane filter. After the filtration, each formulation was filled at 3 mL into 5 cc vials, stoppered and crimped using sterile technique under the clean hood. Two vials of each formulation were placed on a shaker with temperature control. The vials were shaken at 325 RPM at a temperature of 55°C. During the shaking with heat, the general appearance was observed, as described in Example 3.2, periodically over a 42 hour time period. Figures 1 and 2 show the standard and platform formulations, respectively, after 18.5 hours of shaking with heat. The overall appearance results of the shake study indicated that the standard formulation will generate particles over time when subjected to shaking at 55 degree C temperatures more rapidly than the platform formulation.

### Example 3.2: GA, 18.5 hrs Shake study-General Appearance ofatumumab, 20 and 100 mg/mL

General appearance (GA) of an anti-CD20 mab shake study samples is presented in the table below. GA was completed using a general method which can be used for an IgG antibody solution which describes color, clarity and visible particulate matter.

| **Shake Time Point** | **Appearance** |
|---|---|
| **Initial** | |
| Standard | Clear, Colorless, 1-2 Particles present |
| Platform | Clear, Colorless, Particle Free |
| **18.5 hours** | |
| Standard | Clear, Colorless, Several large Particles Present |
| Platform | Clear, Colorless, Particle Free |
| **42 hours** | |
| Standard | Hazy, Colorless, Several Large particles present |
| Platform | Slightly hazy, colorless, particle free |

### Example 4: To determine the thermal stability of ofatumumab solution in the standard and platform buffer by Differential Scanning Calorimetry (DSC).

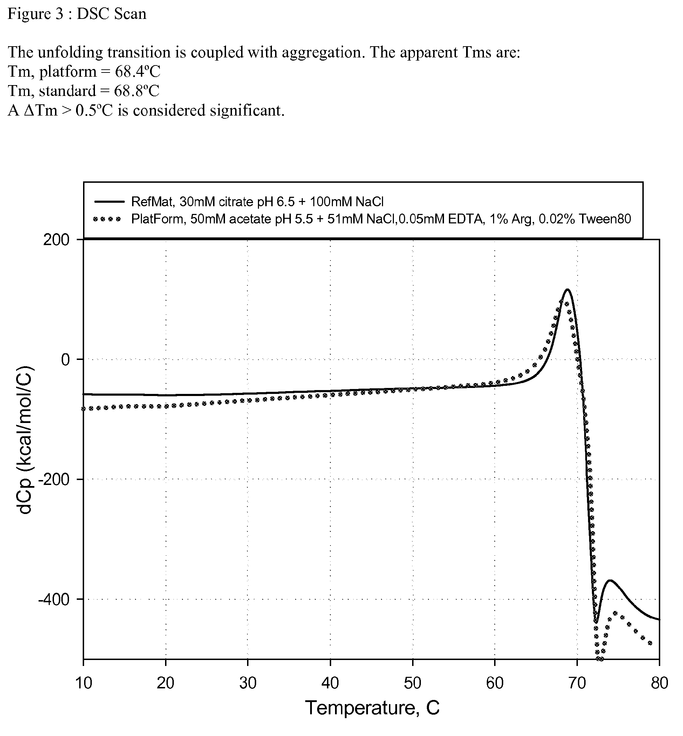

In order to properly complete the testing by DSC, scans of the buffers alone and with protein were acquired. The protein in the standard and platform formulations were diluted to 1 mg/mL as presented in Example 4.1. Data was acquired setting the DSC to scan from 5 - 80°C at a scan rate of 70°C per hour with a 15 minute equilibration before each scan. The volume of the DSC sample cell is ∼ 0.5 mL. After the scans of the buffer and protein were acquired, the buffer scans could then be subtracted from the protein scan. A concentration of the protein in the samples was obtained to correct for the concentration in each scan (See, Example 4.2). The values for Tᵤₙ, °C, start of unfolding , Tₘ, °C, denaturation temperature (at transition maximum) and T_{1/2}, °C, the width of the peak at half-height (reflect changes in tertiary structure and cooperativity of the transitions) were obtained for ofatumumab for each formulation (See, Example 4.3). The actual DSC scans can be seen in Figure 3. Based on the results of the DSC, the ofatumumab in either the standard formulation or the platform formulation had similar DSC profiles and therefore would be expected to have similar thermal stability.

### Example 4.1: Sample preparation for biophysical characterization of ofatumumab pH study:

**1. DILUTIONS**

| **pH** | **Buffer** | **Initial conc. mg/ml** | **Dilute to 1 mg/ml for DSC** | |
|---|---|---|---|---|
| | | | **ml sample** | **ml buffer** |
| *6.5 Standard Formulation* | *30 mM citrate, 100mM NaCl* | *17* | *0.1* | *1.6* |
| *5.5 Platform Formulation* | *50 mM acetate, 51 mM NaCl, 0.05 mM EDTA, 1*% *Arg, 0*.*02*% *Tween-80* | *20* | *0.075* | *1.43* |

### Example 4.2: A280 MEASUREMENTS

| **pH** | **Buffer** | **Initial conc. mg/ml** | **Measured conc. of 0.5 mg/ml dilution** | |
|---|---|---|---|---|
| | | | **mg/ml** | **mM*** |
| *6.5 Standard Formulation* | *30 mM citrate, 100 mM NaCl* | *17* | *0.517* | *0.00345* |
| *5.5 Platform Formulation* | *50 mM acetate, 51 mM NaCl, 0.05 mMEDTA, 1% Arg, 0.02% Tween-80* | *20* | *0.444* | *0.00296* |

| | | | | |
|---|---|---|---|---|
| *use to normalize DSC scans. | | | | |

Prep one sample, blank with corresponding buffer, read 3 times. Use 1 cm cuvette. Subtract A320 absorbance before dividing by extinction coefficient (1.49).

### Example 4.3: DSC results

| **Sample** | **pH** | **Buffer** | **Tᵤₙ, °C** | **Tₘ, °C** | **T_{1/2}, °C** | **Notes** |
|---|---|---|---|---|---|---|
| Standard Formulation | 6.5 | 30 mM citrate, 100 mM NaCl | 62 | 68.8 | 2.9* | |
| Platform Formulation | 5.5 | 50 mM acetate, 51 mM NaCl, 0.05 mM EDTA, 1% Arg, 0.02% Tween-80 | 60 | 68.4 | 3.2* | Similar to Standard Formulation |

| | | | | | | |
|---|---|---|---|---|---|---|
| • * The T_{1/2} values were determined manually. The exothermic contribution from aggregation distorts the baseline, thus these values may be artificially small. | | | | | | |

The anti-CD20 antibody formulation of the present invention can be used to treat a subject with a tumorigenic disorder, e.g., a disorder characterized by the presence of tumor cells expressing CD20 including, for example, B cell lymphoma, e.g., NHL. Examples of tumorigenic diseases which can be treated and/or prevented include B cell lymphoma, e.g., NHL, including precursor B cell lymphoblastic leukemia/lymphoma and mature B cell neoplasms, such as B cell chronic lymhocytic leukemia (CLL)/small lymphocytic lymphoma (SLL), B cell prolymphocytic leukemia, lymphoplasmacytic lymphoma, mantle cell lymphoma (MCL), follicular lymphoma (FL), including low-grade, intermediate-grade and high-grade FL, cutaneous follicle center lymphoma, marginal zone B cell lymphoma (MALT type, nodal and splenic type), hairy cell leukemia, diffuse large B cell lymphoma, Burkitt's lymphoma, plasmacytoma, plasma cell myeloma, post-transplant lymphoproliferative disorder, Waldenstrom's macroglobulinemia, and anaplastic large-cell lymphoma (ALCL).

Further examples of B cell non-Hodgkin's lymphomas are lymphomatoid granulomatosis, primary effusion lymphoma, intravascular large B cell lymphoma, mediastinal large B cell lymphoma, heavy chain diseases (including .gamma., .mu., and .alpha. disease), lymphomas induced by therapy with immunosuppressive agents, such as cyclosporine-induced lymphoma, and methotrexate-induced lymphoma.

An anti-CD20 antibody formulation of the present invention can be used to treat Hodgkin's lymphoma.

Examples of immune disorders (diseases) in which cells expressing CD20 which can be treated and/or prevented by an anti-CD20 antibody formulation of the present invention include autoimmune disorders, such as psoriasis, psoriatic arthritis, dermatitis, systemic scleroderma and sclerosis, inflammatory bowel disease (IBD), Crohn's disease, ulcerative colitis, respiratory distress syndrome, meningitis, encephalitis, uveitis, glomerulonephritis, eczema, asthma, atherosclerosis, leukocyte adhesion deficiency, multiple sclerosis, Raynaud's syndrome, Sjogren's syndrome, juvenile onset diabetes, Reiter's disease, Behcet's disease, immune complex nephritis, IgA nephropathy, IgM polyneuropathies, immune-mediated thrombocytopenias, such as acute idiopathic thrombocytopenic purpura and chronic idiopathic thrombocytopenic purpura, hemolytic anemia, myasthenia gravis, lupus nephritis, systemic lupus erythematosus, rheumatoid arthritis (RA), atopic dermatitis, pemphigus, Graves' disease, Hashimoto's thyroiditis, Wegener's granulomatosis, Omenn's syndrome, chronic renal failure, acute infectious mononucleosis, HIV, and herpes virus associated diseases. Further examples are severe acute respiratory distress syndrome and choreoretinitis. Furthermore, other diseases and disorders include those caused by or mediated by infection of B-cells with virus, such as Epstein-Barr virus (EBV).

Further examples of inflammatory, immune and/or autoimmune disorders in which autoantibodies and/or excessive B lymphocyte activity are prominent and which can be treated and/or prevented by anti-CD20 antibody formulation of the present invention, include the following:
vasculitides and other vessel disorders, such as microscopic polyangiitis, Churg-Strauss syndrome, and other ANCA-associated vasculitides, polyarteritis nodosa, essential cryoglobulinaemic vasculitis, cutaneous leukocytoclastic angiitis, Kawasaki disease, Takayasu arteritis, giant cell arthritis, Henoch-Schonlein purpura, primary or isolated cerebral angiitis, erythema nodosum, thrombangiitis obliterans, thrombotic thrombocytopenic purpura (including hemolytic uremic syndrome), and secondary vasculitides, including cutaneous leukocytoclastic vasculitis (e.g., secondary to hepatitis B, hepatitis C, Waldenstrom's macroglobulinemia, B-cell neoplasias, rheumatoid arthritis, Sjogren's syndrome, or systemic lupus erythematosus); further examples are erythema nodosum, allergic vasculitis, panniculitis, Weber-Christian disease, purpura hyperglobulinaemica, and Buerger's disease; skin disorders, such as contact dermatitis, linear IgA dermatosis, vitiligo, pyoderma gangrenosum, epidermolysis bullosa acquisita, pemphigus vulgaris (including cicatricial pemphigoid and bullous pemphigoid), alopecia areata (including alopecia universalis and alopecia totalis), dermatitis herpetiformis, erythema multiforme, and chronic autoimmune urticaria (including angioneurotic edema and urticarial vasculitis); immune-mediated cytopenias, such as autoimmune neutropenia, and pure red cell aplasia; connective tissue disorders, such as CNS lupus, discoid lupus erythematosus, CREST syndrome, mixed connective tissue disease, polymyositis/dermatomyositis, inclusion body myositis, secondary amyloidosis, cryoglobulinemia type I and type II, fibromyalgia, phospholipid antibody syndrome, secondary hemophilia, relapsing polychondritis, sarcoidosis, stiff man syndrome, and rheumatic fever; a further example is eosinophil fasciitis; arthritides, such as ankylosing spondylitis, juvenile chronic arthritis, adult Still's disease, and SAPHO syndrome; further examples are sacroileitis, reactive arthritis, Still's disease, and gout; hematologic disorders, such as aplastic anemia, primary hemolytic anemia (including cold agglutinin syndrome), hemolytic anemia secondary to CLL or systemic lupus erythematosus; POEMS syndrome, pernicious anemia, and Waldemstrom's purpura hyperglobulinaemica; further examples are agranulocytosis, autoimmune neutropenia, Franklin's disease, Seligmann's disease, .mu.-chain disease, paraneoplastic syndrome secondary to thymoma and lymphomas, and factor VIII inhibitor formation; endocrinopathies, such as polyendocrinopathy, and Addison's disease; further examples are autoimmune hypoglycemia, autoimmune hypothyroidism, autoimmune insulin syndrome, de Quervain's thyroiditis, and insulin receptor antibody-mediated insulin resistance; hepato-gastrointestinal disorders, such as celiac disease, Whipple's disease, primary biliary cirrhosis, chronic active hepatitis, and primary sclerosing cholangiitis; a further example is autoimmune gastritis; nephropathies, such as rapid progressive glomerulonephritis, post-streptococcal nephritis, Goodpasture's syndrome, membranous glomerulonephritis, and cryoglobulinemic nephritis; a further example is minimal change disease; neurological disorders, such as autoimmune neuropathies, mononeuritis multiplex, Lambert-Eaton's myasthenic syndrome, Sydenham's chorea, tabes dorsalis, and Guillain-Barr's syndrome; further examples are myelopathy/tropical spastic paraparesis, myasthenia gravis, acute inflammatory demyelinating polyneuropathy, and chronic inflammatory demyelinating polyneuropathy; cardiac and pulmonary disorders, such as chronic obstructive pulmonary disease (COPD), fibrosing alveolitis, bronchiolitis obliterans, allergic aspergillosis, cystic fibrosis, Loffler's syndrome, myocarditis, and pericarditis; further examples are hypersensitivity pneumonitis, and paraneoplastic syndrome secondary to lung cancer; allergic disorders, such as bronchial asthma and hyper-IgE syndrome; a further example is amaurosis fugax; ophthalmologic disorders, such as idiopathic chorioretinitis; infectious diseases, such as parvovirus B infection (including hands-and-socks syndrome); and gynecological-obstretical disorders, such as recurrent abortion, recurrent fetal loss, and intrauterine growth retardation; a further example is paraneoplastic syndrome secondary to gynaecological neoplasms; male reproductive disorders, such as paraneoplastic syndrome secondary to testicular neoplasms; and transplantation-derived disorders, such as allograft and xenograft rejection, and graft-versus-host disease.

The disease involving cells expressing CD20 is an inflammatory, immune and/or autoimmune disorder selected from ulcerative colitis, Crohn's disease, juvenile onset diabetes, multiple sclerosis, immune-mediated thrombocytopenias, such as acute idiopathic thrombocytopenic purpura and chronic idiopathic thrombocytopenic purpura, hemolytic anemia (including autoimmune hemolytic anemia), myasthenia gravis, systemic sclerosis, and pemphigus vulgaris.

## Claims

1. An anti-CD20 antibody formulation comprising 20-300 mg/mL ofatumumab, wherein the formulation further comprises 10 to 100 mM sodium acetate, 25 to 100 mM sodium chloride, 0.5 to 5% arginine free base, 0.02 to 0.2 mM EDTA, 0.01 to 0.2% polysorbate 80 and adjusted to pH 5.0 to 7.0.

2. The anti-CD20 antibody formulation of Claim 1, wherein the formulation is stable at a temperature of 5°C for at least 2 years.

3. The anti-CD20 antibody formulation of Claim 1, wherein the formulation is stable at a temperature of 25°C for at least 3 months.

4. The anti-CD20 antibody formulation of Claim 1, wherein the formulation is stable at a temperature of 40°C for at least 1 month.

5. The anti-CD20 antibody formulation of Claim 1, wherein the formulation is stable at a temperature of 55°C for at least 1 day.

6. The anti-CD20 antibody formulation of Claim 1, wherein the formulation is stable at a temperature range of 5 to 55°C for at least 1 day with shaking.

7. The anti-CD20 antibody formulation of Claim 1, wherein the sodium acetate is present in an amount of 50mM.

8. The anti-CD20 antibody formulation of Claim 1, wherein the anti-CD20 antibody formulation is pH 5.5.

9. The anti-CD20 antibody formulation of Claim 1, wherein the sodium chloride is present in an amount of 51mM.

10. The anti-CD20 antibody formulation of Claim 1, wherein the arginine free base is present in an amount of 1%.

11. The anti-CD20 antibody formulation of Claim 1, wherein the EDTA is present in an amount of 0.05 mM.

12. The anti-CD20 antibody formulation of Claim 1, wherein the polysorbate 80 is present in an amount of 0.02%.

13. An anti-CD20 antibody formulation comprising ofatumumab in the concentration range of 20-300 mg/mL, wherein the formulation further comprises 50 mM sodium acetate, 51 mM sodium chloride, 1% arginine free base, 0.05 mM EDTA, 0.02% polysorbate 80, and adjusted to pH 5.5.

## Patentansprüche

1. Anti-CD20-Antikörperformulierung, welche 20-300 mg/ml Ofatumumab umfasst, wobei die Formulierung weiterhin 10 bis 100 mM Natriumacetat, 25 bis 100 mM Natriumchlorid, 0,5 bis 5% Arginin freie Base, 0,02 bis 0,2 mM EDTA, 0,01 bis 0,2% Polysorbat 80 umfasst und auf einen pH-Wert von 5,0 bis 7,0 eingestellt ist.

2. Anti-CD20-Antikörperformulierung nach Anspruch 1, wobei die Formulierung bei einer Temperatur von 5°C mindestens 2 Jahre lang stabil ist.

3. Anti-CD20-Antikörperformulierung nach Anspruch 1, wobei die Formulierung bei einer Temperatur von 25°C mindestens 3 Monate lang stabil ist.

4. Anti-CD20-Antikörperformulierung nach Anspruch 1, wobei die Formulierung bei einer Temperatur von 40°C mindestens 1 Monat lang stabil ist.

5. Anti-CD20-Antikörperformulierung nach Anspruch 1, wobei die Formulierung bei einer Temperatur von 55°C mindestens 1 Tag lang stabil ist.

6. Anti-CD20-Antikörperformulierung nach Anspruch 1, wobei die Formulierung bei einer Temperatur im Bereich von 5 bis 55°C unter Schütteln mindestens 1 Tag lang stabil ist.

7. Anti-CD20-Antikörperformulierung nach Anspruch 1, wobei das Natriumacetat in einer Menge von 50 mM vorliegt.

8. Anti-CD20-Antikörperformulierung nach Anspruch 1, wobei die Anti-CD20-Antikörperformulierung einen pH-Wert von 5,5 aufweist.

9. Anti-CD20-Antikörperformulierung nach Anspruch 1, wobei das Natriumchlorid in einer Menge von 51 mM vorliegt.

10. Anti-CD20-Antikörperformulierung nach Anspruch 1, wobei Arginin freie Base in einer Menge von 1% vorliegt.

11. Anti-CD20-Antikörperformulierung nach Anspruch 1, wobei das EDTA in einer Menge von 0,05 mM vorliegt.

12. Anti-CD20-Antikörperformulierung nach Anspruch 1, wobei das Polysorbat 80 in einer Menge von 0,02% vorliegt.

13. Anti-CD20-Antikörperformulierung, welche Ofatumumab im Konzentrationsbereich von 20-300 mg/ml umfasst, wobei die Formulierung weiterhin 50 mM Natriumacetat, 51 mM Natriumchlorid, 1% Arginin freie Base, 0,05 mM EDTA, 0,02% Polysorbat 80 umfasst und auf einen pH-Wert von 5,5 eingestellt ist.

## Revendications

1. Formulation d'anticorps anti-CD20 comprenant 20-300 mg/ml d'ofatumumab, la formulation comprenant en outre de l'acétate de sodium 10 à 100 mM, du chlorure de sodium 25 à 100 mM, 0,5 à 5 % d'arginine base libre, de l'EDTA 0,02 à 0,2 mM, 0,01 à 0,2 % de polysorbate 80, et étant ajustée à pH 5,0 à 7,0.

2. Formulation d'anticorps anti-CD20 selon la revendication 1, la formulation étant stable pendant au moins 2 ans à une température de 5°C.

3. Formulation d'anticorps anti-CD20 selon la revendication 1, la formulation étant stable pendant au moins 3 mois à une température de 25°C.

4. Formulation d'anticorps anti-CD20 selon la revendication 1, la formulation étant stable pendant au moins 1 mois à une température de 40°C.

5. Formulation d'anticorps anti-CD20 selon la revendication 1, la formulation étant stable pendant au moins 1 jour à une température de 55°C.

6. Formulation d'anticorps anti-CD20 selon la revendication 1, la formulation étant stable pendant au moins 1 jour sous agitation sur une plage de températures de 5 à 55°C.

7. Formulation d'anticorps anti-CD20 selon la revendication 1, dans laquelle l'acétate de sodium est présent en une quantité de 50 mM.

8. Formulation d'anticorps anti-CD20 selon la revendication 1, la formulation d'anticorps anti-CD20 ayant un pH de 5,5.

9. Formulation d'anticorps anti-CD20 selon la revendication 1, dans laquelle le chlorure de sodium est présent en une quantité de 51 mM.

10. Formulation d'anticorps anti-CD20 selon la revendication 1, dans laquelle l'arginine base libre est présente en une quantité de 1 %.

11. Formulation d'anticorps anti-CD20 selon la revendication 1, dans laquelle l'EDTA est présent en une quantité de 0,05 mM.

12. Formulation d'anticorps anti-CD20 selon la revendication 1, dans laquelle le polysorbate 80 est présent en une quantité de 0,02 %.

13. Formulation d'anticorps anti-CD20 comprenant de l'ofatumumab à une concentration comprise dans la plage de 20 à 300 mg/ml, la formulation comprenant en outre de l'acétate de sodium 50 mM, du chlorure de sodium 51 mM, 1 % d'arginine base libre, de l'EDTA 0,05 mM, 0,02 % de polysorbate 80, et étant ajustée à pH 5,5.
